(19) Europäisches Patentamt
European Patent Office
Office européen des brevets

(11) Veröffentlichungsnummer: **0 008 039**
**B1**

(12) **EUROPÄISCHE PATENTSCHRIFT**

(45) Veröffentlichungstag der Patentschrift: **01.09.82**

(51) Int. Cl.³: **A 61 K 7/13**

(21) Anmeldenummer: **79102635.4**

(22) Anmeldetag: **25.07.79**

(54) Verwendung eines 6-Amino-3-methyl-phenol enthaltenden Mittels zum Färben von Haaren.

(30) Priorität: **03.08.78 DE 2833989**

(43) Veröffentlichungstag der Anmeldung:
**20.02.80 Patentblatt 80/4**

(45) Bekanntmachung des Hinweises auf die Patenterteilung:
**01.09.82 Patentblatt 82/35**

(84) Benannte Vertragsstaaten:
**AT DE FR GB IT NL**

(56) Entgegenhaltungen:
**DE - A - 2 155 390**
**DE - A - 2 215 303**
**DE - B - 1 143 605**
**NL - A - 7 711 952**

**AMERICAN PERFUMER & AROMATICS, Band
68, August 1956, Seiten 34—37 Pontiac, Ill.
U.S.A. G.S. KASS: "Technology of Modern
Oxidation Hair Dyes, Part II" Seite 35, Tabelle 4,
Verbindungen 17,18**

(73) Patentinhaber: **Wella Aktiengesellschaft
Berliner Allee 65
D-6100 Darmstadt (DE)**

(72) Erfinder: **Konrad, Eugen
Mecklenburger Strasse 101
D-6100 Darmstadt (DE)**
Erfinder: **Mager, Herbert, Dr.
Beaumont 5
CH-1700 Fribourg (CH)**

(56) Entgegenhaltungen:
**BERICHTE DER DEUTSCHEN CHEMISCHEN
GESELLSCHAFT, Band 54B, Nr. 6, 11. Juni
1921, Seiten 1291—1316 Berlin, DE. K.v.
AUWERS et al.: "Uber die Oxydation metasubstituierter o.Amino-phenole" Seiten 1300—
1301: Seite 1314, (Derivate des m-Kresols)—
Seite 1315, dritter Absatz**

## Verwendung eines 6-Amino-3-methyl-phenol enthaltenden Mittels zum Färben von Haaren

Gegenstand der Erfindung ist die Verwendung eines Mittels zum oxidativen Färben von Haaren, welches gekennzeichnet ist durch einen Gehalt an 6 - Amino - 3 - methyl - phenol als Farbkomponente.

Es sind bereits seit längerer Zeit Lösungen von o - Amino - m - kresol bekannt, welche durch Auflösung von 17 g dieser Verbindung unter Erwärmen in 2,5 Liter Wasser und Zusatz von einigen Tropfen einer Lösung von Ammoniumcarbonat erhalten werden (vgl. Berichte der deutschen chemischen Gesellschaft, Band 54 B, Nr. 6, 11. Juni 1921, Seite (1315).

Ferner ist bekannt, daß die isomeren Verbindungen 2 - Amino - 5 - hydroxytoluol und 5 - Amino - 2 - hydroxytoluol imstande sind, Haare anzufärben. Wie dazu beschrieben ist, werden 1 %ige alkalische Lösungen dieser Verbindungen mit Wasserstoffperoxid vermischt und 15 Minuten lang bei 26,7°C F auf die Haare einwirken gelassen. Hierbei werden Haarfärbungen in blonden bzw. bernsteinfarbenen Farbtönen erhalten (vgl. American Perfumer and Aromatics, Band 68, August 1956, Seiten 34/35).

In der Haarfärbung haben Färbemittel auf der Basis von Oxidationsfarbstoffen, welche durch oxidative Kupplung bestimmter Entwicklersubstanzen mit bestimmten Kupplersubstanzen entstehen, eine wesentliche Bedeutung erlangt. Als Entwicklersubstanzen werden insbesondere 2,5 - Diaminotoluol, p - Amino - phenol und 1,4 - Diaminobenzol verwendet. Von den vorzugsweise eingesetzten Kupplersubstanzen kommen Resorcin, 4 - Chlorresorcin, $\alpha$ - Naphthol, m - Aminophenol und Derivative des m-Phenylendiamins wie m - Toluylendiamin und 2,4 - Diaminoanisol in Betracht. Neben diesen genannten Farbvorstufen sind als Bestandteil von Oxidationshaarfärbemitteln weiterhin direkt auf das Haar aufziehende Farbstoffe, insbesondere direktziehende aromatische Nitrofarbstoffe, von Bedeutung. Mit diesen direktziehenden Farbstoffen können Gelb-, Orange-, Rot- und Violettfärbungen erzielt werden.

Farbstoffe, die zur Färbung menschlicher Haare Verwendung finden, müssen zahlreichen Anforderungen gerecht werden. So müssen sie in toxikologischer und dermatologischer Hinsicht unbedenklich sein und Färbungen in der gewünschten Intensität ermöglichen. Außerdem ist es erforderlich, daß durch die Wahl geeigneter Farbvorstufen und geeigneter Direktfarbstoffe eine breite Palette verschiedener Farbnuancen erzeugt werden kann. Weiterhin wird für die erzielten Haarfärbungen eine gute Licht-, Dauerwell-, Säure- und Reibechtheit gefordert. Auf jeden Fall aber müssen solche Haarfärbungen ohne Einwirkung von Licht, Reibung und chemischen Mitteln über einen Zeitraum von mindestens 4 bis 6 Wochen stabil bleiben.

Infolge der Vielzahl der gestellten Anforderungen können die zur Zeit in Oxidationshaarfärbemitteln verwendeten Farbvorstufen und direktziehenden Farbstoffe nicht völlig zufriedenstellen.

Direktziehende aromatische Nitrofarbstoffe führen außerdem bei ihrer Anwendung auf unterschiedlich geschädigtem Haar zu ungleichmäßigen Färbungen. Daher ist eine genügend intensive Färbung von porösen, chemisch geschädigten Haarspitzen oft nicht möglich.

Die Verwendung von o - Aminophenol in Oxidationshaarfärbemitteln ist wegen der nur geringen Farbintensität, welche sich mit dieser Substanz erzielen läßt, lediglich in begrenztem Umfange möglich.

Demgegenüber wurde nun gefunden, daß die Verwendung von Mitteln zum oxidativen Färben von Haaren mit einem Gehalt an 6 - Amino - 3 - methyl - phenol und/oder dessen Salzen mit anorganischen oder organischen Säuren bzw. den aus diesem Phenolderivat mit Alkalilauge gebildeten Phenolaten den erwähnten Anforderungen weitestgehend gerecht werden.

Die erfindungsgemäß zu verwendenden Haarfärbemittel können das 6 - Amino - 3 - methyl - phenol und/oder dessen Salze bzw. Phenolate allein oder auch im Gemisch mit den für die Haarfärbung üblichen Entwickler- und Kupplersubstanzen enthalten. Liegt ein Gemisch vor, so wird das 6 - Amino - 3 - methyl - phenol gegenüber diesen Entwicklerund Kupplersubstanzen im allgemeinen im molaren Unterschuß eingesetzt. In besonderen Fällen kann sich jedoch auch ein äquimolares Verhältnis oder ein diesbezüglicher Überschuß als zweckmäßig erweisen.

Der Gehalt der erfindungsgemäß zu verwendenden Haarfärbemittel an 6 - Amino - 3 - methyl - phenol soll etwa 0,01 bis 2,0 Gewichtsprozent, vorzugsweise 0,02 bis 0,3 Gewichtsprozent, betragen.

Als überliche Entwicklersubstanzen, die in den zu verwendenden Färbemitteln nach vorliegender Anmeldung enthalten sein können, sind insbesondere 1,4-Diaminobenzol, 2,5-Diaminotoluol und p-Aminophenol zu nennen.

Von den üblichen Kupplersubstanzen kommen vorzugsweise $\alpha$-Naphthol, 3,4 - Diaminobenzoesäure, Resorcin, 4 - Chlorresorcin, m - Aminophenol m - Phenylendiamin, m - Toluylendiamin, 2,4 - Diaminoanisol, 2,4 - Diaminobenzylalkohol und 3 - Amino - 6 - methyl - phenyl oder Gemische davon in Betracht.

Zur Erzielung gewisser Farbnuancen können ferner auch übliche direktziehende Farbstoffe, beispielsweise Triphenylmethanfarbstoffe wie Diamond Fuchsin (C.I. 42 510) und Leather Ruby HF (C.I. 42 520), aromatische Nitrofarbstoffe wie 2 - Nitro - 1,4 - diaminobenzol, Azofarbstoffe wie Acid Brown 4 (C.I. 14 805) und Acid Blue 135 (C.I. 13 385), Anthrachinonfarbstoffe wie Disperse Violet 4 (C.I. 61 105), Disperse Blue 1 (C.I. 64 500), Disperse Red 15 (C.I. 60 710), Disperse Violet 1 (C.I. 61 100), außerdem 1,4,5,8 - Tetraaminoanthrachinon und 1,4 - Diamino - anthrachinon enthalten sein.

Die Zubereitungsform der erfindungsgemäß zu verwendenden Haarfärbemittel kann aus einer Lösung, einer Creme, einem Gel oder einer Emulsion bestehen. Ihre Zusammensetzung stellt eine Mischung der Farbkomponenten mit den für solche Zubereitungen üblichen Bestandteilen dar. Als übliche Bestandteile von Cremes, Emulsionen oder Gelen kommen beispielsweise Netzmittel oder Emulgatoren aus den Klassen der anionischen oder nichtionogenen oberflächenaktiven Substanzen wie Fettalkoholsulfate, Fettsäurealkanolamide, Alkylsulfonate, Alkylbenzolsulfonate, oxäthylierte Fettalkohole, oxäthylierte Nonylphenole, ferner Verdicker wie höhere Fettalkohole, Stärke, Cellulosederivate, Paraffinöl und Fettsäuren sowie außerdem Pflegestoffe wie Lanolinderivate, Cholesterin und Pantothensäure in Betracht.

Die erwähnten Bestandteile werden in den für solche Zwecke üblichen Mengen verwendet, z.B. die Netzmittel und Emulgatoren in Konzentrationen von etwa 0,5 bis 30 Gewichtsprozent, während die Verdicker in einer Menge von etwa 0,1 bis 25 Gewichtsprozent in den Zubereitungen enthalten sein können.

Außerdem können in den Haarfärbemitteln noch weitere übliche kosmetische Zusatzstoffe, beispielsweise Antioxidantien wie Ascorbinsäure oder Alkalisulfit, Parfümöle, niedere aliphatische Alkohole wie Äthanol oder Isopropanol, Alkalihydroxide, Komplexbildner und andere vorhanden sein.

Die zu verwendenden Färbemittel nach vorliegender Anmeldung sind, unabhängig von ihrer Zubereitungsform, auf einen pH-Wert im schwach sauren, neutralen oder alkalischen Bereich eingestellt. Insbesondere weisen sie einen pH-Wert im alkalischen Bereich zwischen 8,0 und 11,5 auf, wobei die Einstellung vorzugsweise mit Ammoniak erfolgt. Es können dazu aber auch organische Amine, z.B. Monoäthanolamin oder Triäthanolamin, Verwendung finden.

Das 6 - Amino - 3 - methyl - phenol als wesentlicher Bestandteil der erfindungsgemäßen Haarfärbemittel ergibt bei der Einwirkung eines geeigneten Oxidationsmittels äußerst intensive Gelbfärbungen des Haares. Diesen Färbungen liegt offensichtlich eine Reaktion der genannten Substanz mit sich selbst zugrunde.

Der genannte Oxidationsfarbstoff kann, wie bereits erwähnt, im Gemisch mit überlichen Entwickler- und Kupplerkomponenten eingesetzt werden, wobei er jedoch unter den oxidativen Bedingungen der Haarfärbung mit diesen Komponenten nicht reagiert. Die chemischen Reaktionen zwischen den vorhandenen Entwickler- und Kupplerkomponenten laufen im allgemeinen also unbeeinflußt von dem in den erfindungsgemäßen Haarfärbemitteln enthaltenen 6 - Amino - 3 - methyl - phenol ab. Andererseits wird auch die ablaufende und mit einer Gelbfärbung verbundene chemische Reaktion dieses Phenolderivates mit sich selbst im allgemeinen nicht durch die enthaltenen üblichen Entwickler- und Kupplerkomponenten beeinflußt. Hinsichtlich dieser Unabhängigkeit und Parallelität des Färbevorganges auf der Basis von 6 - Amino - 3 - methyl - phenol ist eine Ähnlichkeit mit Färbungen auf der Basis von direktziehenden aromatischen Nitrofarbstoffen vorhanden.

Durch die Verwendung von 6 - Amino - 3 - methyl - phenol als Bestandteil der erfindungsgemäß zu verwendenden Haarfärbemittel besteht nun die Möglichkeit, die mit nachteiligen Eigenschaften behafteten direktziehenden gelb und orange färbenden aromatischen Nitrofarbstoffe unter gleichzeitiger Verbesserung der Färbeeigenschaften zu ersetzen. Dadurch ist es ebenfalls möglich, nun auch poröse, chemisch geschädigte Haarspitzen intensiv einzufärben.

Auch in toxikologischer und dermatologischer Hinsicht stellt die Verwendung von 6 - Amino - 3 - methyl - phenol einen wesentlichen Fortschritt dar, wie sich beispielsweise bei dessen Einsatz an Stelle des üblichen 4 - Nitro - 1,2 - diaminobenzols zeigt. So wirkt die genannte Nitroverbindung sensibilisierend und besitzt außerdem eine relativ hohe akute Toxizität, während das Phenolderivat gemäß vorliegender Erfindung diese Nachteile nicht aufweist. Darüber hinaus ermöglicht es die starke Färbekraft des 6 - Amino - 3 - methylphenols, daß diese Substanz in sehr niedriger Konzentration in den erfindungsgemäß zu verwendenden Haarfärbemitteln eingesetzt werden kann.

Die Anwendung der nach vorliegender Anmeldung zu verwendenden Haarfärbemittel erfolgt in bekannter Weise, indem man sie kurz vor dem Gebrauch mit einem Oxidationsmittel vermischt und das Gemisch auf das Haar aufträgt. Als Oxidationsmittel zur Entwicklung der Haarfärbung kommt insbesondere Wasserstoffperoxyd, beispielsweise als 6 %ige Lösung, bzw. dessen Additionsverbindungen an Harnstoff, Melamin oder Natriumborat in Betracht.

Die Anwendungstemperaturen liegen im Bereich von 15 bis 50°C. Nach einer Einwirkungszeit von etwa 15 bis 50 Minuten, vorzugsweise etwa 30 Minuten, wird das Haar mit Wasser ausgespült und getrocknet. Gegebenenfalls wird im Anschluß an diese Spülung mit einem Shampoo gewaschen und mit einer schwachen organischen Säure, wie beispielsweise Zitronensäure oder Weinsäure, nachgespült.

Als Bestandteil der erfindungsgemäß zu verwendenden Haarfärbemittel eignet sich das 6 - Amino - 3 - methyl - phenol insbesondere als Nuancierfarbstoff zur Erzielung von Naturtönen, modischen Farbtönen und besonders matten Farbnuancen. In diesem Zusammenhang ist ferner von Bedeutung, daß die durch das 6 - Amino - 3 - methyl - phenol erhaltenen Gelbtöne bei gleichzeitiger Anwesenheit des isomeren 6 - Methyl - 3 - amino - phenols eine Farbverschiebung nach Orange erfahren.

Die nachstehenden Beispiele sollen den Gegenstand der Erfindung näher erläutern.

Beispiele

Beispiel 1  Haarfärbelösung

    0,3 g  6-Amino-3-methyl-phenol
    10,0 g  Laurylalkohol-diglykoläthersulfat,
            28 %ige wäßrige Lösung
    10,0 g  Isopropanol
    0,1 g  Natriumhydroxid, fest
    0,3 g  Natriumsulfit, wasserfrei
    10,0 g  Ammoniak, 22 %ig
    69,3 g  Wasser
    _____
    100,0 g

50 g des vorstehenden Haarfärbemittels werden kurz vor dem Gebrauch mit 50 ml Wasserstoffperoxidlösung (6 %ig) vermischt. Anschließend trägt man das Gemisch auf blonde Naturhaare auf und läßt 30 Minuten lang bei 40° C einwirken. Das Haar hat eine farbsatte, leuchtend gelbe Färbung erhalten.

Beispiel 2  Haarfärbemittel in Gelform

    0,3 g  6-Amino-3-methyl-phenol
    0,2 g  1,4-Diaminobenzol
    15,0 g  Ölsäure
    7,0 g  Isopropanol
    0,1 g  Natriumhydroxid, fest
    0,3 g  Ascorbinsäure
    10,0 g  Ammoniak, 22 %ig
    67,1 g  Wasser
    _____
    100,0 g

50 g des obigen Haarfärbemittels werden kurz vor dem Gebrauch mit 50 ml Wasserstoffperoxidlösung (6 %ig) gemischt und das Gemisch anschließend auf hellblonde, teilweise ergraute Naturhaare aufgetragen. Nach einer Einwirkungszeit von 30 Minuten bei 40°C wird mit Wasser gespült und getrocknet. Das Haar hat einen gleichmäßigen blonden, natürlichen Farbton erhalten.

Beispiel 3  Haarfärbemittel in Cremeform

    0,25 g  6-Amino-3-methyl-phenol
    0,30 g  3-Amino-6-methyl-phenol
    0,05 g  1,4-Diaminobenzol
    3,50 g  Laurylalkohol-diglykoläthersulfat,
            28 %ige wäßrige Lösung
    15,00 g  Cetylalkohol
    0,20 g  Natriumhydroxid, fest
    0,30 g  Natriumsulfit, wasserfrei
    10,00 g  Ammoniak, 22 %ig
    70,40 g  Wasser
    _____
    100,00 g

Man vermischt kurz vor dem Gebrauch 50 g dieses Haarfärbemittels mit 50 ml Wasserstoffperoxidlösung (6 %ig) und läßt das Gemisch 30 Minuten lang bei 40°C auf blonde Naturhaare einwirken. Danach wird mit Wasser gespült und getrocknet. Das Haar ist in einem intensiven violettstichigen Rotton gefärbt.

Alle in der vorliegenden Anmeldung angegebenen Prozentzahlen stellen Gewichtsprozente dar.

**Patentansprüche**

1. Verwendung eines Mittels zum oxidativen Färben von Haaren, dadurch gekennzeichnet, daß es als Farbkomponente 6 - Amino - 3 - methyl - phenol enthält.

2. Verwendung eines Mittels nach Anspruch 1, dadurch gekennzeichnet, daß es das 6 - Amino - 3 - methyl - phenol in einer Konzentration von etwa 0,01 bis 2,0 Gewichtsprozent, vorzugsweise 0,02 bis 0,3 Gewichtsprozent, enthält.

3. Verwendung eines Mittels nach Anspruch 1 und 2, dadurch gekennzeichnet, daß es das 6 - Amino - 3 - methyl - phenol in Form eines Salzes mit einer anorganischen oder organischen Säure bzw. in Form des Phenolates enthält.

4. Verwendung eines Mittels nach Anspruch 1 bis 3, dadurch gekennzeichnet, daß es übliche Entwickler- und Kupplersubstanzen sowie direktziehende Farbstoffe enthält.

5. Verwendung eines Mittels nach Anspruch 1 bis 4, dadurch gekennzeichnet, daß es mindestens eine der Entwicklersubstanzen 1,4 - Diaminobenzol, 2,5 - Diaminotoluol und p - Aminophenol enthält.

6. Verwendung eines Mittels nach Anspruch 1 bis 5, dadurch gekennzeichnet, daß es mindestens eine der Kupplersubstanzen α - Naphthol, 3,4 - Diaminobenzoesäure, Resorcin, 4 - Chlorresorcin, m - Aminophenol, m - Phenylendiamin, m - Toluylendiamin, 2,4 - Diaminoanisol, 2,4 - Diaminobenzylalkohol und 3 - Amino - 6 - methyl - phenol enthält.

7. Verwendung eines Mittels nach Anspruch 1 bis 6, dadurch gekennzeichnet, daß es mindestens einen der direktziehenden Farbstoffe Diamond Fuchsin (C.I. 42 510), Leather Ruby HF (C.I. 42 520), 2 - Nitro - 1,4 - diaminobenzol, Acid Brown 4 (C.I. 14 805), Acid Blue 135 (C.I. 13 385), Disperse Violet 4 (C.I. 61 105), Disperse Blue 1 (C.I. 64 500), Disperse Red 15 (C.I. 60 710), Disperse Violet 1 (C.I. 61 100), 1,4,5,8 - Tetraamino - anthrachinon und 1,4 - Diamino - anthrachinon enthält.

8. Verwendung eines Mittels nach Anspruch 1 bis 7, dadurch gekennzeichnet, daß es zusätzlich Antioxidantien, vorzugsweise Ascorbinsäure oder Natriumsulfit, enthält.

9. Verwendung eines Mittels nach Anspruch 1 bis 8, dadurch gekennzeichnet, daß es zusätzlich Netzmittel, Emulgatoren und/oder Verdicker enthält.

## Claims

1. Use of a composition for the oxidative colouring of hair, characterised in that it contains as colouring component 6 - amino - 3 - methyl - phenol.

2. Use of a composition according to Claim 1, characterised in that it contains 6 - amino - 3 - methyl - phenol in a concentration from about 0.01 to 2.0 weight percent, preferably 0.02 to 0.3 weight percent.

3. Use of composition according to either of claims 1 and 2, characterised in that it contains 6 - amino - 3 - methyl - phenol in the form of a salt with an inorganic or organic acid or in the form of a phenolate.

4. Use of a composition according to any of claims 1 to 3, characterised in that it contains common developer and coupler substances as well as direct dyes.

5. Use of a composition according to any of claims 1 to 4, characterised in that it contains at least one of the developer substances 1,4 - diaminobenzene, 2,5 - diaminotoluene and p-aminophenol.

6. Use of a composition according to any of claims 1 to 5, characterised in that it contains at least one of the coupler substances $\alpha$ - Naphthol, 3,4 - diaminobenzoic acid, resorcinol, 4 - chlorresorcinol, m - aminophenol, m - phenylendiamine, m - toluylendiamine, 2,4 - diaminoanisole, 2,4 - diaminobenzyl alcohol and 3 - amino - 6 - methyl - phenol.

7. Use of a composition according to any of claims 1 to 6, characterised in that it contains at least one of the direct dyes Diamond Fuchsin (C.I. 42 510), Leather Ruby HF (C.I. 42 520), 2 - nitro - 1,4 - diaminobenzene; Acid Brown 4 (C.I. 14 805); Acid Blue 135 (C.I. 13 385); Disperse Violet 4 (C.I. 61 105); Disperse Blue 1 (C.I. 64 500); Disperse Red 15 (C.I. 60 710); Disperse Violet 1 (C.I. 61 100); 1, 4, 5, 8 - tetraamino - anthraquinone and 1,4 - diamino - anthraquinone.

8. Use of a composition according to any of claims 1 to 7, characterised in that it contains additional antioxidants, preferably ascorbic acid or sodium sulfite.

9. Use of a composition according to any of claims 1 to 8, characterised in that it contains additional wetting agents, emulsifiers and/or thickeners.

## Revendications

1. Utilisation d'une composition pour le teinture par oxydation des cheveux, caractérisée en ce qu'elle renferme comme agent colorant du 6 - amino - 3 - méthyl - phénol.

2. Utilisation d'une composition suivant la revendication 1, caractérisée en ce qu'elle renferme du 6 - amino - 3 - méthyl - phénol selon une concentration d'environ 0,01 à 2,0 % en poids et de préférence de 0,02 à 0,3 % en poids.

3. Utilisation d'une composition suivant les revendications 1 et 2, caractérisée en ce qu'elle renferme le 6 - amino - 3 - méthyl - phénol sous forme de son sel avec un acide inorganique ou organique ou bien sous forme du phénolate.

4. Utilisation d'une composition suivant les revendications 1 à 3, caractérisée en ce qu'elle renferme des substances de développement et de couplage ou de condensation usuelles, ainsi que des colorants à fixation directe.

5. Utilisation d'une composition suivant les revendications 1 à 4, caractérisée en ce qu'elle renferme au moins l'une des substances de développement formées par le 1,4 - diaminobenzène, le 2,5 - diaminotoluène et le p - aminophénol.

6. Utilisation d'une composition suivant les revendications 1 à 5, caractérisée en ce qu'elle renferme au moins une des substances de condensation formées par l'$\alpha$ - naphtène, l'acide 3,4 - diaminobenzoïque, la résorcine, la 4 - chlororésorcine, le m - aminophénol, la m - phénylènediamine, la m - toluylènediamine, le 2,4 - diaminoanisol, l'alcohol 2,4 - diaminobenzylique et le 3 - amino - 6 - méthyl - phénol.

7. Utilisation d'une composition suivant les revendications 1 à 6, caractérisée en ce qu'elle renferme l'un au moins des colorants à fixation directe ci-après: Diamond Fuchsin (C.I. 42 510), Leather Ruby HF (C.I. 42 520), 2 - nitro - 1,4 - diaminobenzène, Acid Brown 4 (C.I. 14 805), Acid Blue 135 (C.I. 13 385), Disperse Violet 4 (C.I. 61 105), Disperse Blue 1 (C.I. 64 500), Disperse Red 15 (C.I. 60 710), Disperse Violet 1 (C.I. 61 100), 1, 4, 5, 8 - tétraamino - anthraquinone et 1,4 - diamino - anthraquinone.

8. Utilisation d'une composition suivant les revendications 1 à 7, caractérisée en ce qu'elle renferme en outre des agents antioxydants, de préferénce de l'acide ascorbique ou du sulfite de sodium.

9. Utilisation d'une composition suivant les revendications 1 à 8, caractérisée en ce qu'elle renferme en outre des agents mouillants, des émulsionnants et (ou) des épaississants.